(19) Europäisches Patentamt
European Patent Office
Office européen des brevets

(11) **EP 3 935 193 B1**

(12) **EUROPEAN PATENT SPECIFICATION**

(45) Date of publication and mention
of the grant of the patent:
**22.07.2026 Bulletin 2026/30**

(21) Application number: **20707151.5**

(22) Date of filing: **05.03.2020**

(51) International Patent Classification (IPC):
***C12Q 1/6886*** *(2018.01)*

(52) Cooperative Patent Classification (CPC):
**C12Q 1/6886;** C12Q 2600/156

(86) International application number:
**PCT/EP2020/055915**

(87) International publication number:
**WO 2020/178400 (10.09.2020 Gazette 2020/37)**

(54) **METHOD TO DIAGNOSE A CMMRD**

VERFAHREN ZUR DIAGNOSE EINER CMMRD

PROCÉDÉ POUR DIAGNOSTIQUER LE SYNDROME CMMRD

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.03.2019 EP 19305261**

(43) Date of publication of application:
**12.01.2022 Bulletin 2022/02**

(73) Proprietors:
• **INSERM (Institut National de la Santé
et de la Recherche Médicale)
75013 Paris (FR)**
• **Assistance Publique-Hôpitaux de Paris (APHP)
75004 Paris (FR)**
• **Sorbonne Université
75006 Paris (FR)**

(72) Inventors:
• **DUVAL, Alex
75012 Paris (FR)**
• **COULET, Florence
75013 Paris (FR)**
• **GUILLERM, Erell
75013 Paris (FR)**
• **BUHARD, Olivier
75012 Paris (FR)**

(74) Representative: **Icosa
83 avenue Denfert-Rochereau
75014 Paris (FR)**

(56) References cited:
WO-A1-2016/127067    WO-A1-2018/137678
WO-A1-2019/024598    CN-A- 109 182 525

**Description**

**FIELD OF THE INVENTION:**

The present invention relates a method of diagnosing a CMMRD cancer or a MSI leukemia/lymphoma in a patient in need thereof.

**BACKGROUND OF THE INVENTION:**

Lynch syndrome (LS) is a hereditary cancer syndrome caused by heterozygous germline mutations in mismatch repair (MMR) genes (i.e., MLH1, MSH2, MSH6, or PMS2). Constitutional MMR deficiency (CMMRD) is a distinct and rare inherited cancer syndrome (Online Mendelian Inheritance in Man [OMIM] database accession no. 276300) observed in patients with biallelic germline MMR pathogenic variants. Whereas MMR-deficient (dMMR) cancers in LS patients arise mostly during adulthood, CMMRD is characterized by the development of multiple MMR-deficient tumors during childhood, including early-onset colorectal cancers, lymphoma/leukemia, and brain tumors (1,2). These tumors are usually very aggressive, highlighting the need for rapid detection of CMMRD in order to adopt the appropriate treatment and surveillance strategies for CMMRD children and their families (3). As could be expected, the high mutation and neoantigen loads that characterize MMR-deficient neoplasias were recently associated with durable response to immune checkpoint inhibition in CMMRD patients (4,5).

CMMRD is difficult to diagnose for several reasons. First is the broad spectrum of tumors seen in this rare cancer syndrome and the lack of clear, disease-specific clinical features. In this regard, we recently proposed a scoring system for the diagnosis of suspected CMMRD 2. Second is the detection of variants with unknown functional significance (VUS) in around 30% of patients that leads to non-informative results (6).

**SUMMARY OF THE INVENTION:**

In the present work, the inventors aimed to develop a test that could drastically simplify and improve the diagnosis of CMMRD based on DNA sequence analysis of primary blood cells (PBCs) from patients. Using massive parallel sequencing, they explored the possibility that MSI, the main genomic and functional consequence of constitutive MMR-deficiency, was likely to occur in CMMRD PBCs well before any transformation.

Thus, the present invention relates to a method of diagnosing a CMMRD cancer or a MSI leukemia/lymphoma in a patient in need thereof. Particularly, the invention is defined by its claims.

**DETAILED DESCRIPTION OF THE INVENTION:**

A first aspect of the invention relates to a method of diagnosing a CMMRD cancer or a MSI leukemia/lymphoma in a patient in need thereof comprising i) extracting DNA from a sample obtained from said patient ii) sequencing a number (N) of repeat sequences having a length of (x) nucleic acids from the DNA of said patient, iii) repeating the steps i) and ii) for at least one control subject having stable microsatellite cancer (MSS control subject), iv) doing a log 10 transformation of the reads counts per locus for said patient and for the MSS control subject(s), and doing a limit regression for each repeat obtained from the MSS control subject(s), and v) obtaining the ms.score by doing the following formula :

$$\sum_{n=1}^{N} \Delta_n$$

wherein, N = number maximal of repeat sequenced; n = number of repeat sequences, $\Delta$ = number of reads (patient in need thereof sample) - number of reads (limit regression from the MSS control subject(s)) and vi) comparing the ms.score obtained with the patient in need thereof with the ms.score of the MSS control subject(s) and vii) concluding that the patient in need thereof has a CMMRD cancer or a MSI leukemia/lymphoma when his ms.score is superior than the ms.score of the MSS control subject(s).

As used herein the limit-regression is used here for calibration/reference for the patient tested. According to the invention, the limit regression for each repeat obtained from the MSS control subject is done by taking the lowest correlation coefficient and regression slope.

According to the invention, the limit-regression obtained for each repeat obtained from the MSS control subject(s) is used as a model to predict log 10 transformed reads counts that a patient would exceed if he has a CMMRD or a MSI leukemia/lymphoma. According to the invention, if there are more than one MSS control subject used, the limit-regression obtained will be a global limit-regression for all the MSS control(s) subject(s).

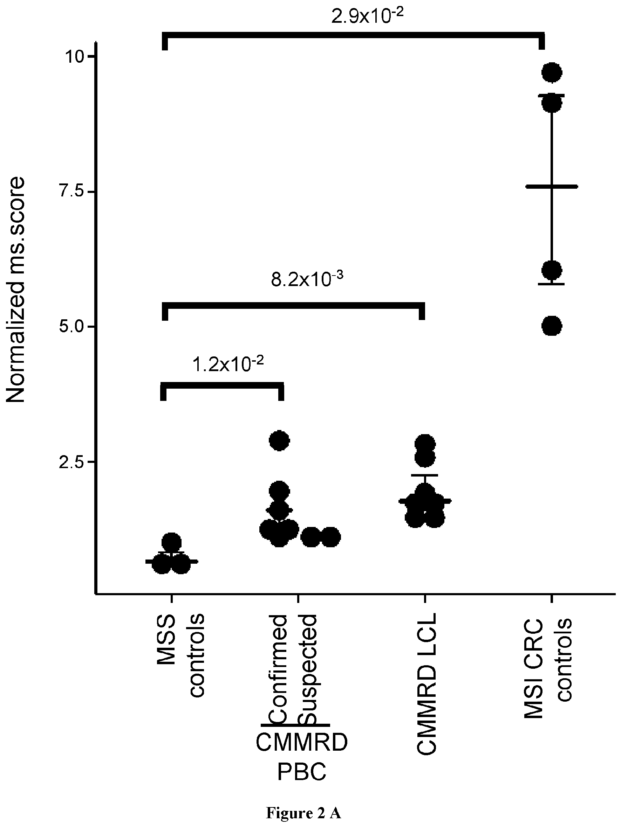

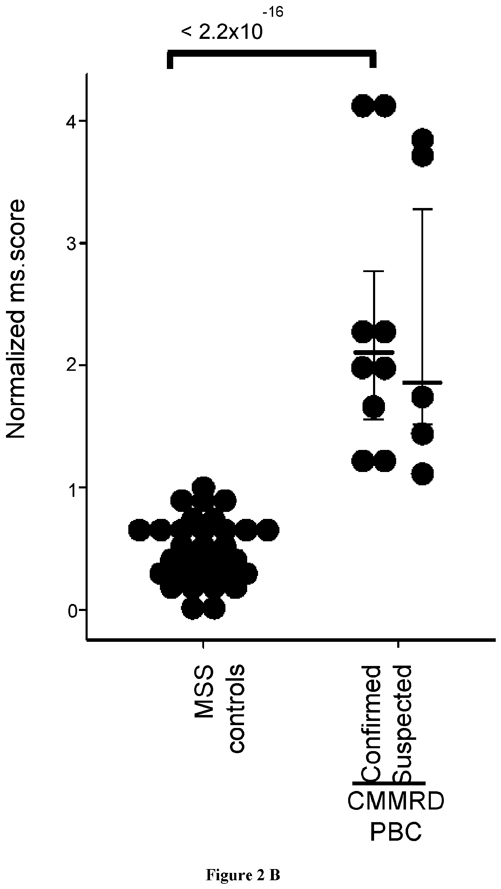

Thus, when using this method (log 10 for all samples (patient and MSS control(s)) and limit-regression for MSS control(s)), and when rescaling the ms.score of patients and MSS controls subjects to the highest ms.score of the MSS controls, the ms.score will be superior to 1 (see figure 2 for example) when the patient will have a CMMRD or a MSI leukemia/lymphoma.

Thus, another embodiment of the invention relates to a method of diagnosing a CMMRD cancer or a MSI leukemia/lymphoma in a patient in need thereof comprising i) extracting DNA from a sample obtained from said patient ii) sequencing a number (N) of repeat sequences having a length of (x) nucleic acids from the DNA of said patient, iii) repeating the steps i) and ii) for at least one MSS control subject, iv) doing a log 10 transformation of the reads counts per locus for said patient, and doing a log 10 transformation of the reads counts per locus and a limit regression for each repeat obtained from the MSS control subject(s), and v) obtaining the ms.score by doing the following formula :

$$\sum_{n=1}^{N} \Delta_n$$

wherein, N = number maximal of repeat sequenced; n = number of repeat sequences, $\Delta$ = number of reads (patient in need thereof sample) - number of reads (limit regression from the MSS control subject(s)) and vi) comparing the ms.score obtained with the patient in need thereof with the ms.score of the MSS control subject(s) and vii) concluding that the patient in need thereof has a CMMRD cancer or a MSI leukemia/lymphoma when his ms.score is superior than the ms.score of the MSS control subject(s).

Thus, in still another embodiment of the invention relates to a method of diagnosing a CMMRD cancer or a MSI leukemia/lymphoma in a patient in need thereof comprising i) extracting DNA from a sample obtained from said patient ii) sequencing a number (N) of repeat sequences having a length of (x) nucleic acids from the DNA of said patient, iii) repeating the steps i) and ii) for at least one MSS control subject, iv) doing a log 10 transformation of the reads counts per locus for the patient, and doing a log 10 transformation of the reads counts per locus and a limit regression for each repeat obtained from the MSS control subject(s) by taking the lowest correlation coefficient and regression slope, and using this limit-regression as a model to predict log 10 transformed reads counts that a patient would exceed if he has a CMMRD or MSI leukemia/lymphoma, and v) obtaining the ms.score by doing the following formula :

$$\sum_{n=1}^{N} \Delta_n$$

wherein, N = number maximal of repeat sequenced; n = number of repeat sequences, $\Delta$ = number of reads (patient in need thereof sample) - number of reads (limit regression from the MSS control subject(s)) and vi) comparing the ms.score obtained with the patient in need thereof with the ms.score of the MSS control subject(s) and vii) concluding that the patient in need thereof has a CMMRD cancer when his ms.score is superior than the ms.score of the MSS control subject(s).

As use herein, the term "CMMRD" for "Constitutional Mismatch Repair Deficiency" denotes a hereditary cancer predisposition that typically presents in infancy, childhood or young adulthood. Individuals with CMMRD are at risk for developing hematologic malignancies, brain tumors, colon, small bowel, uterine, gastric, urologic and other types of cancer. Individuals with CMMRD have an estimated a 16-fold increased risk for persons with biallelic MSH2 mutations. They can have multiple diagnoses of these cancers throughout their life. Additionally, "café au lait" macules have been reported in these individuals. CMMRD is caused by mutations in the Mismatch Repair (MMR) genes: MLH1, MSH2, MSH6 and PMS2. When a person has one mutation in one of their MMR gene then they have Lynch syndrome. A person must have 2 gene mutations in the same MMR gene to develop CMMRD.

As used herein, the term "MSI cancer" denotes that an instability is detected in at least 2 microsatellite markers. On the contrary, if instability is detected in one or no microsatellite marker, then said cancer is a "MSS cancer" This definition is valuable only if the diagnostic is done by the pentaplex method (see for example Suraweera N et al., Evaluation of tumor microsatellite instability using five quasimonomorphic mononucleotide repeats and pentaplex PCR. Gastroenterology. 2002 or Buhard O et al., Multipopulation analysis of polymorphisms in five mononucleotide repeats used to determine the microsatellite instability status of human tumors. J Clin Oncol.2006). A "MSS cancer" denotes to a cancer having stable microsatellite. A "MSI cancer" refers to a cancer having microsatellite instable.

As used herein, the term "repeat" denotes the number of nucleic acids (or nucleic bases) repeated for a specific locus. So the term "repeat" denotes a length of nucleic acids. For example, if the repeat is 12 for the nucleic acids A, this means that the nucleic acids A is repeated 12 time consecutively in a specific locus. According to the invention, as used herein, the term "repeat" as the same meaning than "microsatellite".

As used herein, the term "the number of repeat" or "reads" denotes the number of time when a "repeat" of a specific length (for example 12 nucleic acids A) for a specific locus is repeated. Thus, "the number of repeat" also denotes the number of loci containing a given repeat.

As used the term "read" denotes a DNA fragment produced by a sequencer instrument which are exact copies of the locus to be sequenced and are used to determine the content and sequential order of its nucleic acids.

As used herein, the term "N" denotes the number maximal of repeat of one specific sequencing assay which also corresponds to the number of loci tested.

As used herein the term "sample" refers to any biological sample obtained from the subject that is liable to contain DNA and particularly germinal DNA. Typically, samples include but are not limited to body fluid samples, such as blood, plasma or serum. In a particular embodiment, the sample is the peripheral blood mononuclear cell (PBMC), primary blood cells (PBCs) or tumor circulating lymphoid cells. In a particular embodiment, germinal DNA obtained from PBMCs or PBCs will be used to diagnose CMMRD. In a particular embodiment, the invention will be also useful for the diagnostic of MSI leukemia/lymphoma when the sample is cancerous circulating lymphoid cells and when the DNA from these cancerous cells is sequenced.

As used herein the term a "MSI leukemia/lymphoma" denotes all leukemia and lymphoma with microsatellites instability.

As used herein, the term "patient" denotes a mammal. Typically, a patient according to the invention refers to any subject (preferably human) afflicted with a CMMRD cancer or MSI leukemia/lymphoma cancer. The term "MSS control subject" also refers to a subject having a MSS cancer. According to the invention, the number of MSS control subject used can be from 1 to 30 or more. Accordingly, several MSS control subject can be used to do a limit regression according to the method of the invention.

As used herein the term "nucleic acid" or "nucleic base" has its general meaning in the art and refers to refers to a coding or non-coding nucleic sequence. Nucleic acids include DNA (deoxyribonucleic acid) and RNA (ribonucleic acid) nucleic acids. Example of nucleic acid thus include but are not limited to DNA, mRNA, tRNA, rRNA, tmRNA, miRNA, piRNA, snoRNA, and snRNA. Nucleic acids thus encompass coding and non-coding region of a genome (i.e. nuclear or mitochondrial).

In a particular embodiment, the sequencing according to the invention is done to a number of loci between 10 and 10000, particularly, between 100 and 10000 and more particularly between 100 and 1000.

In a particular embodiment, the reads counts per locus after sequencing is between 500 and 4000, particularly between 1000 and 4000, particularly between 1000 and 3000 and more particularly between 1500 and 2500.

In a particular embodiment, the lengths (x) (or number) of nucleic acids in a specific repeat is between 8 and 30 and particularly 8 and 14. According to the invention the lengths (x) (or number) of nucleic acids in a specific repeat can be, 8, 9, 10, 11, 12, 13, 14, 15, 16, 17, 18, 19, 20, 21, 22, 23, 24, 25, 26, 27, 28, 29 or 30.

As used herein, the term "cancer" has its general meaning in the art and includes, but is not limited to, solid tumors and blood borne tumors. The term cancer includes diseases of the skin, tissues, organs, bone, cartilage, blood and vessels. The term "cancer" further encompasses both primary and metastatic cancers. Examples of cancers include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma;

amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In some embodiments, the subject suffers from a colorectal cancer, more particularly a metastatic colorectal cancer.

In a particularly embodiment, the cancer is a colorectal cancer and thus a CMMRD colorectal cancer.

In one embodiment, a step of viii) communicating the result to the patient may be added to the method of the invention.

In a particular embodiment, the method as described above allows to distinguish a MSS cancer to a CMMRD cancer.

The ms.score of the present invention can be performed by one or more programmable processors executing one or more computer programs to perform functions by operating on input data and generating output. The algorithm can also be performed by, and apparatus can also be implemented as, special purpose logic circuitry, e.g., an FPGA (field programmable gate array) or an ASIC (application-specific integrated circuit). Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry. To provide for interaction with a user, embodiments of the invention can be implemented on a computer having a display device, e.g., in non-limiting examples, a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. Accordingly, in some embodiments, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the invention, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

***Sequencing methods:***

According to the invention, the sequencing step may be accomplished by any method, including without limitation chemical sequencing, using the Maxam-Gilbert method (Methods in Enzymology 65, 499-560 (1980)); by enzymatic sequencing, using the Sanger method Proc. Natl. Acad. Sci. USA 74, 5463-67 (1977)).; mass spectrometry sequencing; sequencing using a chip-based technology; and real-time quantitative PCR.

In the chemical sequencing, base specific modifications result in a base specific cleavage of the radioactive or fluorescently labeled DNA fragment. With the four separate base specific cleavage reactions, four sets of nested fragments are produced which are separated according to length by polyacrylamide gel electrophoresis (PAGE). After autoradiography, the sequence can be read directly since each band (fragment) in the gel originates from a base specific cleavage event. Thus, the fragment lengths in the four "ladders" directly translate into a specific position in the DNA sequence.

In the enzymatic sequencing, the four base specific sets of DNA fragments are formed by starting with a primer/template system elongating the primer into the unknown DNA sequence area and thereby copying the template and synthesizing a complementary strand by DNA polymerases, such as Klenow fragment of E. coli DNA polymerase I, a DNA polymerase from Therm us aquaticus, Taq DNA polymerase, or a modified T7 DNA polymerase, Sequenase (Tabor et al., Proc. Natl. Acad. Scl. USA 84, 4767-4771 (1987)), in the presence of chain-terminating reagents.

Several new methods for DNA sequencing (High-throughput sequencing (HTS) methods) were developed in the mid to late 1990s and were implemented in commercial DNA sequencers by the year 2000. Together these were called the "next-generation" or "second-generation" sequencing methods. These HTS included but are not limited to: Single-molecule real-time sequencing, Ion semiconductor, Pyrosequencing, Sequencing by synthesis, Sequencing by ligation, Nanopore Sequencing, Chain termination and Sequencing by hybridization. Some of these methods allow a Whole Gene Sequencing (WGS), Whole Exome Sequencing (WES) or a Targeted Sequencing.

In a particular embodiment, the sequencing according to the method of the invention is an ultra-deep sequencing like Second-Generation Sequencing (NGS), performed using targeted massive parallel sequencing approcah, by the mean of which a specified panel of regions in the genome, herein mononucleoid microsatellites, are sequenced (see for example Goodwin, S and all, 2016. Coming of age: Ten years of next-generation sequencing technologies. Nature Reviews Genetics).

Further methods which are not part of the invention

A patient identified as having a CMMRD or a MSI leukemia/lymphoma cancer can be treated with radiotherapy, chemotherapy, immunotherapy or a combination thereof .

The term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin;

podophyllinic acid; 2-ethylhydrazide; procarbazine; PSK®; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.].) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; capecitabine; and phannaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and phannaceutically acceptable salts, acids or derivatives of any of the above.

When it is concluded that the subject has a CMMRD cancer then the physician can take the choice to administer the patient with a targeted therapy.

Targeted cancer therapies are drugs or other substances that block the growth and spread of cancer by interfering with specific molecules ("molecular targets") that are involved in the growth, progression, and spread of cancer. Targeted cancer therapies are sometimes called "molecularly targeted drugs," "molecularly targeted therapies," "precision medicines," or similar names.

In some embodiments, the targeted therapy consists of administering the subject with a tyrosine kinase inhibitor. The term "tyrosine kinase inhibitor" refers to any of a variety of therapeutic agents or drugs that act as selective or non-selective inhibitors of receptor and/or non-receptor tyrosine kinases. Tyrosine kinase inhibitors and related compounds are well known in the art and described in U.S Patent Publication 2007/0254295, It will be appreciated by one of skill in the art that a compound related to a tyrosine kinase inhibitor will recapitulate the effect of the tyrosine kinase inhibitor, e.g., the related compound will act on a different member of the tyrosine kinase signaling pathway to produce the same effect as would a tyrosine kinase inhibitor of that tyrosine kinase. Examples of tyrosine kinase inhibitors and related compounds suitable for use in methods of embodiments of the present invention include, but are not limited to, dasatinib (BMS-354825), PP2, BEZ235, saracatinib, gefitinib (Iressa), sunitinib (Sutent; SU11248), erlotinib (Tarceva; OSI-1774), lapatinib (GW572016; GW2016), canertinib (CI 1033), semaxinib (SU5416), vatalanib (PTK787/ZK222584), sorafenib (BAY 43-9006), imatinib (Gleevec; STI571), leflunomide (SU101), vandetanib (Zactima; ZD6474), MK-2206 (8-[4-aminocyclobutyl)phenyl]-9-phenyl-1,2,4-triazolo[3,4-f][1,6]naphthyridin-3(2H)-one hydrochloride) derivatives thereof, analogs thereof, and combinations thereof. Additional tyrosine kinase inhibitors and related compounds suitable for use in the present invention are described in, for example, U.S Patent Publication 2007/0254295, U.S. Pat. Nos. 5,618,829, 5,639,757, 5,728,868, 5,804,396, 6,100,254, 6,127,374, 6,245,759, 6,306,874, 6,313,138, 6,316,444, 6,329,380, 6,344,459, 6,420,382, 6,479,512, 6,498,165, 6,544,988, 6,562,818, 6,586,423, 6,586,424, 6,740,665, 6,794,393, 6,875,767, 6,927,293, and 6,958,340, In certain embodiments, the tyrosine kinase inhibitor is a small molecule kinase inhibitor that has been orally administered and that has been the subject of at least one Phase I clinical trial, more preferably at least one Phase II clinical, even more preferably at least one Phase III clinical trial, and most preferably approved by the FDA for at least one hematological or oncological indication. Examples of such inhibitors include, but are not limited to, Gefitinib, Erlotinib, Lapatinib, Canertinib, BMS-599626 (AC-480), Neratinib, KRN-633, CEP-11981, Imatinib, Nilotinib, Dasatinib, AZM-475271, CP-724714, TAK-165, Sunitinib, Vatalanib, CP-547632, Vandetanib, Bosutinib, Lestaurtinib, Tandutinib, Midostaurin, Enzastaurin, AEE-788, Pazopanib, Axitinib, Motasenib, OSI-930, Cediranib, KRN-951, Dovitinib, Seliciclib, SNS-032, PD-0332991, MKC-I (Ro-317453; R-440), Sorafenib, ABT-869, Brivanib (BMS-582664), SU-14813, Telatinib, SU-6668, (TSU-68), L-21649, MLN-8054, AEW-541, and PD-0325901.

When it is concluded that the subject has a CMMRD cancer then the physician can take the choice to administer the subject with an immunotherapeutic agent.

The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively the immunotherapeutic treatment may consist of administering the subject with an amount of immune cells (T cells, NK, cells, dendritic cells, B cells...).

Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents.

Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors.

Interferons (IFNs) contemplated by the present invention include the common types of IFNs, IFN-alpha (IFN-a), IFN-beta (IFN-beta) and IFN-gamma (IFN-y). IFNs can act directly on cancer cells, for example, by slowing their growth, promoting their development into cells with more normal behaviour and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). The use of IFN-alpha, alone or in combination with other immunotherapeutics or with chemotherapeutics, has shown efficacy in the treatment of various cancers including melanoma (including metastatic melanoma), renal cancer (including metastatic renal cancer), breast cancer, prostate cancer, and cervical cancer (including metastatic cervical cancer).

Interleukins contemplated by the present invention include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present invention. Interleukins, alone or in combination with other immunotherapeutics or with chemotherapeutics, have shown efficacy in the treatment of various cancers including renal cancer (including metastatic renal cancer), melanoma (including metastatic melanoma), ovarian cancer (including recurrent ovarian cancer), cervical cancer (including metastatic cervical cancer), breast cancer, colorectal cancer, lung cancer, brain cancer, and prostate cancer.

Interleukins have also shown good activity in combination with IFN-alpha in the treatment of various cancers (Negrier et al., Ann Oncol. 2002 13(9):1460-8; Touranietal, J. Clin. Oncol. 2003 21(21):398794).

Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in subjects undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Colony stimulating factors have shown efficacy in the treatment of cancer, including melanoma, colorectal cancer (including metastatic colorectal cancer), and lung cancer.

Non-cytokine adjuvants suitable for use in the combinations of the present invention include, but are not limited to, Levamisole, alum hydroxide (alum), Calmette-Guerin bacillus (ACG), incomplete Freund's Adjuvant (IFA), QS-21, DETOX, Keyhole limpet hemocyanin (KLH) and dinitrophenyl (DNP). Non-cytokine adjuvants in combination with other immuno- and/or chemotherapeutics have demonstrated efficacy against various cancers including, for example, colon cancer and colorectal cancer (Levimasole); melanoma (BCG and QS-21); renal cancer and bladder cancer (BCG).

In addition to having specific or non-specific targets, immunotherapeutic agents can be active, i.e. stimulate the body's own immune response, or they can be passive, i.e. comprise immune system components that were generated external to the body.

Passive specific immunotherapy typically involves the use of one or more monoclonal antibodies that are specific for a particular antigen found on the surface of a cancer cell or that are specific for a particular cell growth factor. Monoclonal antibodies may be used in the treatment of cancer in a number of ways, for example, to enhance a subject's immune response to a specific type of cancer, to interfere with the growth of cancer cells by targeting specific cell growth factors, such as those involved in angiogenesis, or by enhancing the delivery of other anticancer agents to cancer cells when linked or conjugated to agents such as chemotherapeutic agents, radioactive particles or toxins.

Monoclonal antibodies currently used as cancer immunotherapeutic agents that are suitable for inclusion in the combinations of the present invention include, but are not limited to, rituximab (Rituxan®), trastuzumab (Herceptin®),

ibritumomab tiuxetan (Zevalin®), tositumomab (Bexxar®), cetuximab (C-225, Erbitux®), bevacizumab (Avastin®), gemtuzumab ozogamicin (Mylotarg®), alemtuzumab (Campath®), and BL22. Monoclonal antibodies are used in the treatment of a wide range of cancers including breast cancer (including advanced metastatic breast cancer), colorectal cancer (including advanced and/or metastatic colorectal cancer), ovarian cancer, lung cancer, prostate cancer, cervical cancer, melanoma and brain tumours. Other examples include anti-CTLA4 antibodies (e.g. Ipilimumab), anti-PD1 antibodies, anti-PDL1 antibodies, anti-TIMP3 antibodies, anti-LAG3 antibodies, anti-B7H3 antibodies, anti-B7H4 antibodies or anti-B7H6 antibodies.

Particularly, a patient diagnosed as having a CMMRD or a MSI leukemia/lymphoma according to the invention can be treated by immunotherapy like immune checkpoint blockade involving anti-CTLA4, anti-PD1, anti-PD-L1 alone or in combination, or anti-cancer vaccines or dendritic cells vaccines based on tumour specific antigens.

Active specific immunotherapy typically involves the use of cancer vaccines. Cancer vaccines have been developed that comprise whole cancer cells, parts of cancer cells or one or more antigens derived from cancer cells. Cancer vaccines, alone or in combination with one or more immuno- or chemotherapeutic agents are being investigated in the treatment of several types of cancer including melanoma, renal cancer, ovarian cancer, breast cancer, colorectal cancer, and lung cancer. Non-specific immunotherapeutics are useful in combination with cancer vaccines in order to enhance the body's immune response.

The immunotherapeutic treatment may consist of an adoptive immunotherapy as described by Nicholas P. Restifo, Mark E. Dudley and Steven A. Rosenberg "Adoptive immunotherapy for cancer: harnessing the T cell response, Nature Reviews Immunology, Volume 12, April 2012). In adoptive immunotherapy, the subject's circulating lymphocytes, or tumor infiltrated lymphocytes, are isolated in vitro, activated by lymphokines such as IL-2 or transuded with genes for tumor necrosis, and readministered (Rosenberg et al., 1988; 1989). The activated lymphocytes are most preferably be the subject's own cells that were earlier isolated from a blood or tumor sample and activated (or "expanded") in vitro. This form of immunotherapy has produced several cases of regression of melanoma and renal carcinoma.

When it is concluded that the subject has a CMMRD cancer then the physician can take the choice to administer the subject with a radiotherapeutic agent.

The term "radiotherapeutic agent" as used herein, is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

The invention will be further illustrated by the following figures and examples. However, these examples and figures should not be interpreted in any way as limiting the scope of the present invention.

## FIGURES:

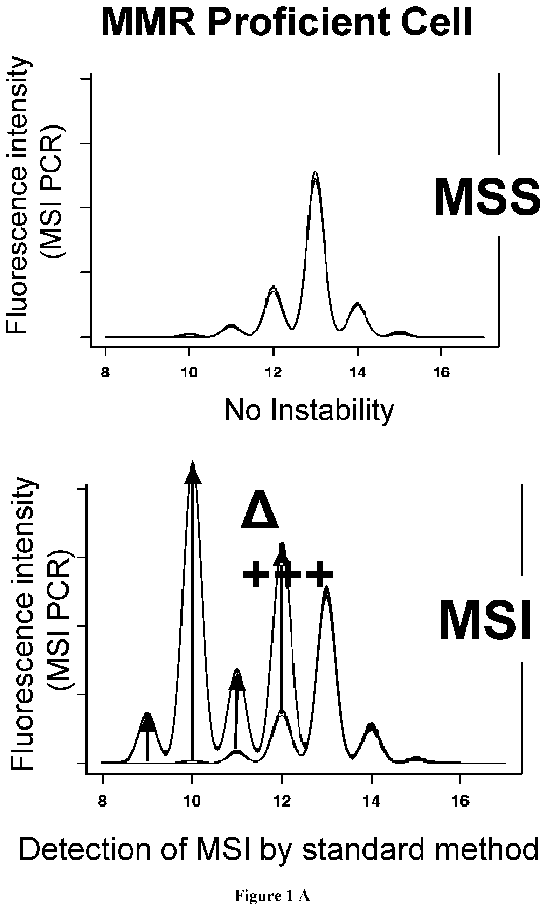

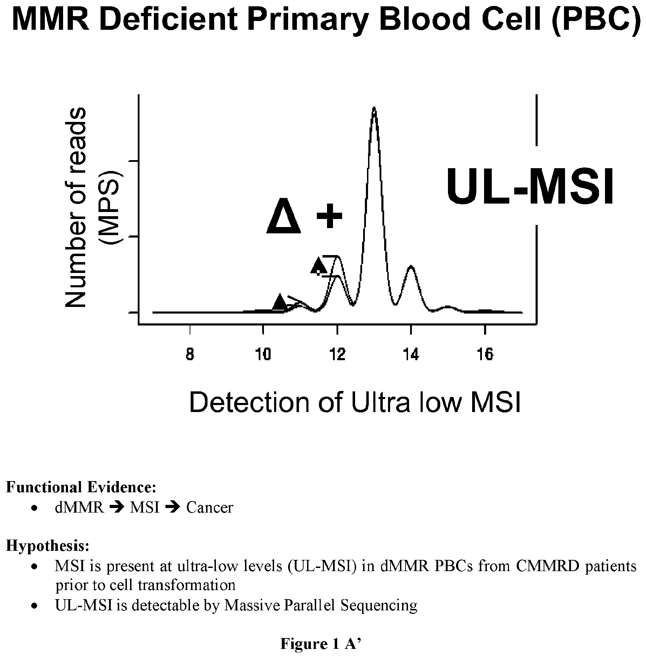

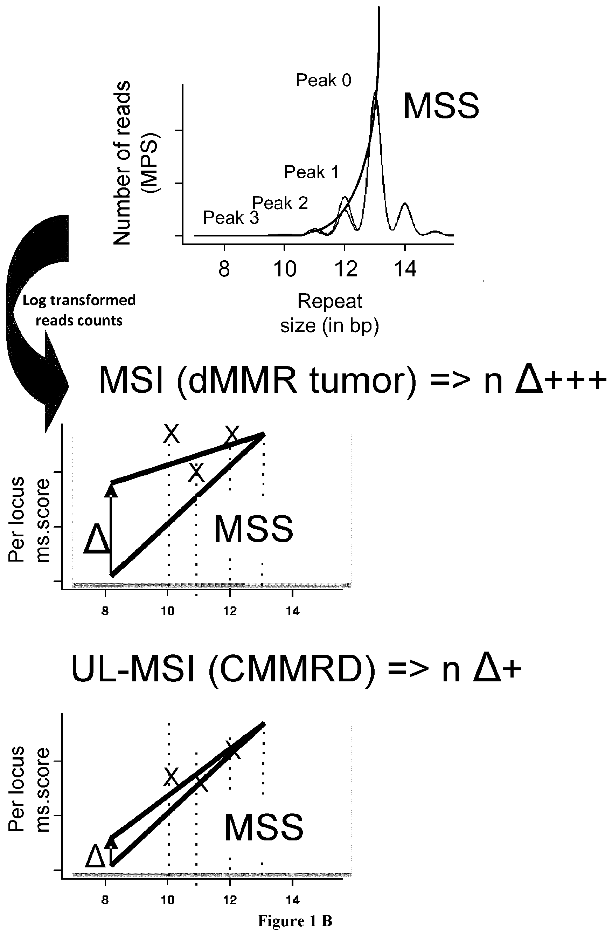

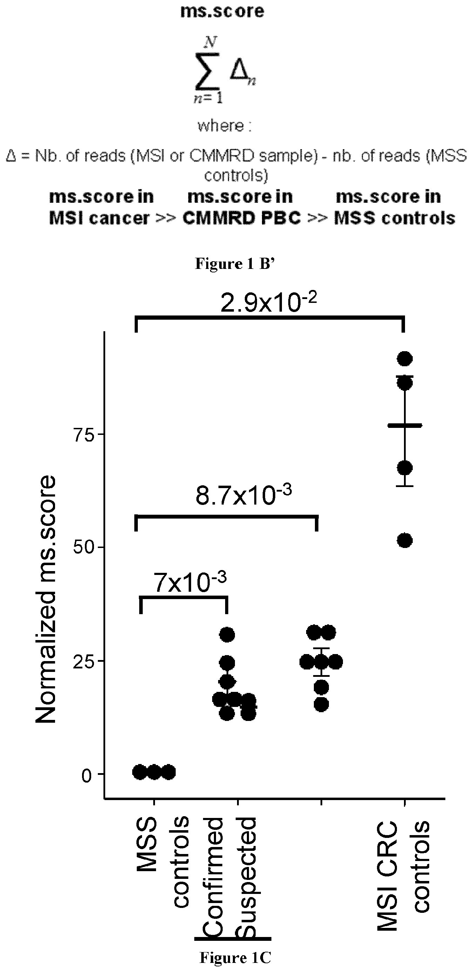

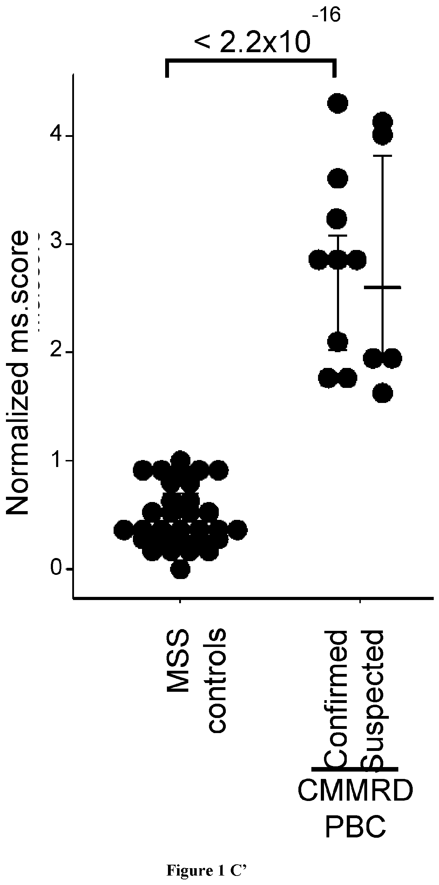

**Figure 1:** A and A') Working hypothesis: microsatellite mutations are observed using standard PCR methods and analysis of fragment length size in mononucleotide microsatellites in the tumor DNA of MMR-deficient (dMMR)/MSI colorectal cancers. We hypothesized that PBCs from CMMRD patients acquire microsatellite mutations at ultra low levels (UL-MSI) even before cancer onset and that such mutations can be detected using massive parallel sequencing. B and B') Principle of the algorithm used to detect MSI and UL-MSI in dMMR tumors and CMMRD PBCs, respectively: read counts of stutters based on the major allele of mononucleotide loci follow a log 10 distribution. After determination of the limit regression parameters from MSS controls (i.e. regression parameters nearest to those of an MSI sample) to establish an empiric standard model per locus, the observed read counts are compared to those predicted by the models and the differences are summed for all loci in each sample to derive the ms.score. C and C') Classification of discovery set and validation set samples based on their ms.score.. The normalized ms.score for each sample is plotted for MSS controls, PBC from CMMRD patients, CMMRD LCLs, and MSI CRC controls. Blue streacks identify CMMRD cases common to the two studies. The error bars represent the 1st and 3rd quartiles and the median of each distribution. Brackets indicate the p-values obtained by comparing each distribution to MSS controls using a Spearman correlation test (10000 permutations). MPS: Massive Parallel Sequencing.

**Figure 2:** Classification of samples tested in the 2 studies, after determination of their ms.score using the full panel (number of loci is indicated for each study). The dots represent each sample plotted after scaling of the ms.score. CMMRD dots identify samples which are common to both studies. In the discovery study (panel A) as anticipated, MSI tumors had the highest ms.score, CMMRD LCLs had intermediate ms.score values and CMMRD PBCs approximately same level values for ms.score. p-values for distribution comparison are indicated (Spearman Correlation test, 10000 permutations). The error bars represent 1st and 3rd quartile distribution and the median of each distribution. In the validation study (panel B), cases present systematically with higher ms.score when compared to controls, thus confirming the interest of this metric to evaluate UL-MSI in CMMRD PBC samples.

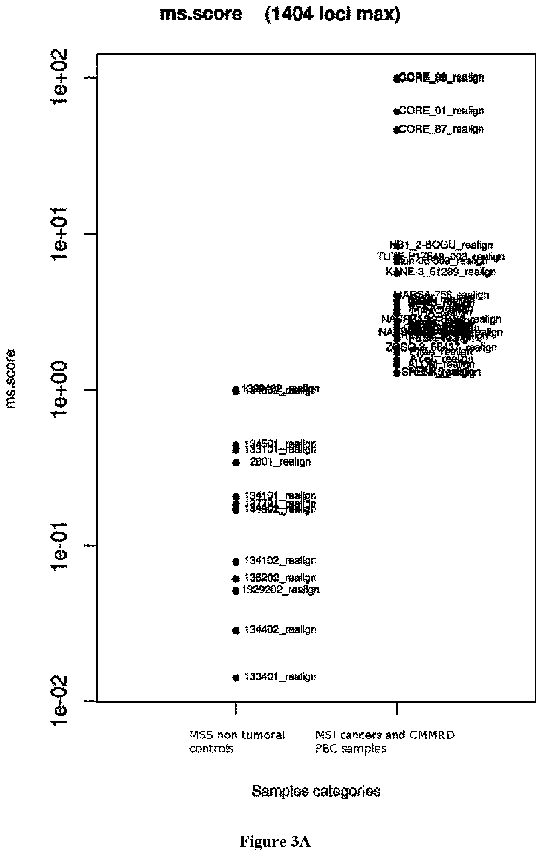

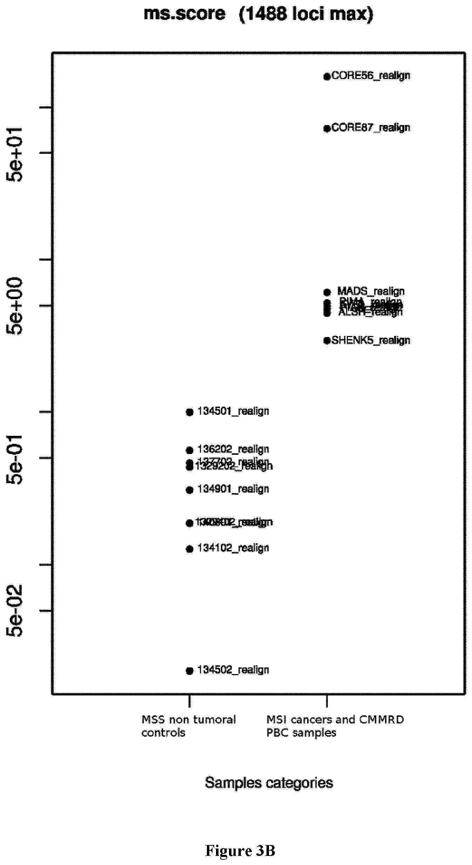

**Figure 3:** Classification CMMRD and MSI tumour samples in 2 assays after determination of their ms.score using the full panel (number of loci is indicated for each assays). A. The first assay is realized with 15 non-tumoral MSS control samples, 27 CMMRD blood samples and 4 MSI colon cancer. B. The second assay is realized with 10 non-tumoral MSS control samples, 6 CMMRD samples and 2 MSI colon cancer samples.

**Table 1**: description of the 16 CMMRD patients included in this study

| Sample ID | Gender | Age at first tumor | Clinical score[a] | Germline MMR genetic status[b] | Allelic status[c] | Diagnostic according to MMR genetics | Funtional MMR Testing[d] | gMSI Testing[e] |
|---|---|---|---|---|---|---|---|---|
| **CMMRD patients with confirmed molecular diagnosis, i.e. with biallelic pathogenic MMR gene alterations** | | | | | | | | |
| C01.2 | F | 20 | 7 | PMS2, | cpd | CMMRD | + | NA |
| C02 | F | 4 | 3 | PMS2, | HMZ | CMMRD | + | NI |
| C04 | F | 4 | 7 | PMS2, | cpd | CMMRD | + | + |
| C06.2 | M | 5 | 7 | PMS2, | HMZ | CMMRD | + | + |
| C09.1 | M | 4 | 5 | PMS2, | HMZ | CMMRD | + | + |
| C10.1 | M | 6 | 4 | PMS2, | HMZ | CMMRD | + | + |
| C12 | M | 2 | 5 | MSH6, | HMZ | CMMRD | + | - |
| C13.1 | M | 10 | 10 | MSH6, | cpd | CMMRD | + | - |
| C14.1 | F | 9 | 8 | MSH6, | cpd | CMMRD | + | NI |
| C15 | M | 5 | 10 | MLH1, | HMZ | CMMRD | + | + |
| C29.1 | M | 6 | 4 | PMS2, | HMZ | CMMRD | + | + |
| **patients with a strong suspicion of CMMRD, i.e. with a clinical score greater or equal to 3** | | | | | | | | |
| C20.1 | F | 9 | 7 | MSH6, | cpd | inconclusive | + | - |
| C20.2 | F | 6 | 7 | MSH6, | cpd | inconclusive | + | - |
| C21 | M | 14 | 14 | MSH6, PV/VUS | cpd HTZ, | inconclusive | Doubtful | - |
| C22 | F | 16 | 8 | MSH6, VUS/VUS | HMZ, HTZ | inconclusive | + | - |
| C23 | F | 6 | 13 | MSH6, | HMZ | inconclusive | Doubtful | - |

a) Clinical score according to Wimmer 2014 (2); b) PV: pathogenic variant, VUS: variant of unknown significance; c) HMZ: homozygous, HTZ: heterozygous, cpd HTZ: compound heterozygous; d) +: case with ev-MSI alterations and resistance to methylating agents, doubtful: test failure for one condition, according to Bodo 2015 (6); e) +: altered gMSI profile, -: no alteration, NI: not informative, NA: not available, according to Ingham 2013 (10)

**EXAMPLE:**

**Material & Methods**

**Sequencing and data analysis**

After samples sequencing, the read count distribution was determined for all loci and an ms.score was obtained by integrating differences between the distribution observed for read counts of test samples and the distribution of read counts predicted by a standard model derived from MSS controls (see Fig. 1A and 1B for a full description of the working hypothesis and principle of the ms.score method). The ms.score was rescaled based on the highest ms.score of the MSS control subjects so that control MSS cases did not exceed 1, but MSI was greater than 1. Read count distribution analyses and ms.score determinations were made using the R base package. Distribution of ms.scores was compared between confirmed CMMRD patients and controls using a Spearman correlation test with 10000 permutations (see also Supplementary Material and Methods for further details and Supplementary tools for the scripts used for ms.score determination).

### Study populations

Eligible subjects included 16 patients either already diagnosed with CMMRD (i.e., with biallelic deleterious germline mutations in one of the 4 major MMR genes, N=11) or with a strong clinical suspicion of CMMRD (i.e., a clinical score ≥ 3 according to prior criteria from the C4CMMRD consortium, N=5) (2). Thirty-four MMR-proficient subjects considered free of MMR germline defects were used as MSS (non-MSI) controls. MSI controls consisted of colorectal cancer samples (CRCs, N=4) and lymphoblastoid cell lines (LCLs) obtained from CMMRD patients (N=7) and previously shown to exhibit an unequivocal MSI phenotype following in vitro culture 2. All patients gave written informed consent and institutional review boards/ethics committees of the participating centers approved this study.

### Mutation screening of MMR genes and other functional tests to assist CMMR diagnosis

All analyses were performed in clinically approved laboratories. Analysis of the MLH1, MSH2, and MSH6 genes was performed in different laboratories whereas analysis of PMS2 was performed in the Rouen, Lille, or Innsbruck laboratories, as previously described. LCLs were obtained following standard Epstein-Barr virus infection. Ex vivo MSI analysis and Methylation Tolerance assay were performed with LCLs as previously reported (6).

### Study design

The above samples (16 cases with confirmed or strong clinical suspicion of CMMRD CMMRD, 11 MSI controls and 34 MSS controls) were distributed into two partially overlapping sets, i.e. a discovery set (14 control cases including 7 CMMRD LCLs, 4 MSI CRC and 3 MSS controls; 8 cases with confirmed or strong clinical suspicion of CMMRD), and a validation set (31 MSS healthy control cases; 14 cases with confirmed or strong clinical suspicion of CMMRD, of which 6 were also included in the discovery set). The two sets were analyzed separately by massive parallel sequencing with two partially overlapping panels of probes covering mononucleotide repeats between 6 to 27 base pairs in length (2586 regions of interest (ROIs) and 1187 ROIs for discovery and validation studies respectively, 395 ROIs common to the two sets). The first set was designed to allow a theoretical depth of coverage of 10000X using the sequencing settings using already explored and validated ROIs for MSI analysis (7,8). For the second study, we desired theoretical depth of coverage was 6000X, thus allowing 48 DNAs to be included, using probes targeting loci issued from the same studies a the first set. A panel of only 3 MSS cases was used in the discovery study, as this was sufficient to build a working algorithm for MSI/UL-MSI identification. At least 30 MSS cases was considered sufficient to allow for polymorphisms and to obtain accurate models of the loci analysed in the validation study (32 MSS DNAs initially processed, 1 failed library QC). The 16 CMMRD cases initially processed were randomly drawn from the consortium database to avoid selection bias (16 confirmed or suspected CMMRD cases initially, 2 failed library QC).

### Massive parallel sequencing of MNR loci

Samples were sequenced and analysed separately. After DNA sonication and quality control, barcoded DNA libraries were obtained using Nimblegen SeqCap solutions, pooled and targeted fragments were captured with SeqCap EZ Choice library kit from Nimblegen. Sequencing was performed on a NextSeq 500 (Illumina). Reads were trimmed and examined for their quality and depth of coverage. Per sample mean depth of coverage ranged from 1778 to 6140, with on average 242 uncovered loci for the discovery study. In the second study, per sample mean depth of coverage ranged from 235 to 5349, with no more than 33 uncovered probes for all but 1 sample. Globally, this second assay delivered less good quality reads with a less homogeneous coverage among loci when considering samples individually and between-sample global mean coverage, with a higher level of PCR/sequencing duplicates. Furthermore, one sample (C01.2) had a low mean coverage (82X) and a higher number of uncovered loci (n=485). It was however tested as for all other samples in order to evaluate the classification algorithm in the situation of a low number of qualified reads.

Fastq files were processed for global alignment against hg19 reference genome, then a more accurate local alignment of mononucleotide repeats containing reads was performed with the GATK IndelRealigner tool to minimize misalignments. Counts for reads covering integrally the targeted mononucleotide repeats were obtained with a modified version of MSIsensor (11) and further processed to evaluate the presence of aberrant mononucleotide repeat sequences in the samples.

**Determination of analytical readout to classify MSI/UL-MSI cases**

For each sample, per locus distribution was normalized to the major allele count, log-transformed: a linear regression model was applied for each MSS controls locus. Limit regression parameters (i.e. minimum slope, maximum y-intercept and coefficient of correlation r) were retained to generate a locus-limit model, which could thus be considered as background models for the distribution of stutters based on the major allele. Only repeats presenting with the same nucleotide length to that of the reference genome sequence were retained. After removal of polymorphic alleles (considered when another proximal peak presented a frequency exceeding 0.7) and artefactual counts (reads with noise and/or interrupted repeats), the models were then used to predict log values of read counts for stutters of each major allele of a standard MSS sample. As MSI frequently leads to the shortening mononucleotide repeats, a positive difference between observed read counts and predictions would be observed in MSI samples but not in MSS. A per locus ms.score was obtained by integrating differences between observed read count and prediction. Per locus ms.scores were then summed up to obtain the sample's ms.score, using it as a summary metric for accumulated alterations in all repeats analysed in a specific sample. Ms.score was then normalised in each study so that MSS controls had an ms.score not exceeding 1: a test sample should then present with a value greater than 1 in order to be classified as MSI/UL-MSI.

For the discovery study, the 3 MSS controls samples were used to generate the standardised regression models for each locus of the panel and the predicted log-transformed read counts values for an MSS standard.

In the second study, the 31 MSS controls available after sequencing were used to generate the standardised regression models and the predicted log-transformed read counts values for a MSS standard.

**Optimized probes set: Application of Molecular Tests for Detection of Confirmed and Suspected CMMRD Individuals**

In the two studies, we tried to improve the ms.score determination by limiting the number of ROIs. Most informative probes were selected using two parameters: per locus mean depth of coverage over samples and ms.score value distribution. Mean depth of coverage for each locus averaged on the 22 samples of the first study was higher than 2000 reads in 88% of all analysed loci. Selecting loci covered from 1000 to 5000 reads, by windows of 1000X each selection, did not significantly change the relative values of ms.score when compared between the different sample classes (data not shown), even though the absolute ms.score value dropped when lowering the number of incorporated loci. This was anticipated since very low numbers of loci causes a significant loss of information, leading to a poorer performance (low sensitivity and specificity). Even though mononucleotide repeat size could represent a effective parameter to probe selection, no difference was evidenced for repeats of at least 8 bp (data not shown).

When keeping only the loci with a raw ms.score exceeding a determined threshold (i.e. 0.1) for a minimum number of samples (i.e. n>16), only 118 loci were necessary to discriminate MSS controls from CMMRD cases or MSI positive controls as accurately as the full panel. Using this subset of loci, the ms.score for the MSS controls ranged from 0 to 1 (mean=0.47), whereas the ms.score for CMMRD PBC ranged from 12.77 to 30.74 (mean=20.21) and from 13.98 to 16.16 (mean=15.07) for confirmed and suspected cases of CMMRD respectively (MSS controls vs all CMMRD PBCs, $P=2.4 \times 10^{-5}$; data not shown).

In the case-control study, mean depth of coverage for each locus averaged on the 45 samples was higher than 2000 reads in 89% of all analysed loci. Selecting loci covered from 1000 to 5000 reads, by windows of 1000X each selection, did not change significantly the relative values of the ms.score when compared between the different sample classes (Fig. S3A), but extreme coverage categories (i.e. <1000X and >=5000X) showed poorer performance, probably due to reads artefacts accumulation and/or PCR/sequencing duplication level. Mononucleotide repeat size did not affect significantly the classification of the tested samples, as anticipated from the previous results (data not shown).

When keeping only the loci with a raw ms.score exceeding the same threshold (i.e. 0.1) for a minimum number of samples (i.e. n>5), only 18 loci could discriminate MSS controls from CMMRD cases as accurately as the full panel. The ms.score for the MSS controls ranged from 0 to 1 (mean=0.49), whereas the ms.score for CMMRD PBC ranged from 1.74 to 4.30 (mean=2.77) and from 1.62 to 4.13 (mean=2.81) for confirmed and suspected CMMRD cases respectively (MSS controls vs all CMMRD PBCs, $P=2.1 \times 10^{-7}$) (data not shown).

**Statistical analysis**

Read counts distribution analyses and ms.score determination were made using the R base package. The comparison between sample and control ms.score distributions was calculated using an unilateral Welch two-sample test.

Scripts written (bash and R) to generate the analyses were developed under Ubuntu 16.04 environment and are available in supplementary tools.

## Results

### Discovery Set: Proof-of-concept study

To test our working hypothesis, summarized in Figure 1A, we first investigated whether UL-MSI could be detected by massive parallel sequencing using the ms.score in PBCs from confirmed CMMRD subjects (N=6) as compared to the 3 MSS controls and MSI controls (4 MSI CRC samples and 7 CMMRD LCLs) included in the discovery set. The ms.score values for MSS controls ranged from 0.56 to 1, with a mean of 0.74. The ms.score values for CMMRD LCLs ranged from 1.40 to 2.82 (mean=1.96), while for CMMRD PBCs they ranged from 1.13 to 2.89 (mean=1.67) for confirmed cases, thus showing a sensitivity of 100% (Figure 2A). They ranged from 1.07 to 1.11 (mean=1.09) for suspected CMMRD cases. Comparison of the ms.score distribution between the different groups showed a significant difference between MSS and MSI controls (CRCs, $P$=0.029; CMMRD LCLs, $P$=0.0082), but also with confirmed CMMRD PBCs ($P$=0.012). Interestingly, UL-MSI was also detected in suspected CMMRD cases, a result supporting the diagnosis already obtained by the functional tests and by their high clinical score. Critical inputs for the algorithms were the number of loci and the depth of coverage (data not shown). Significant differences between the ms.score of confirmed CMMRD and MSS control samples were observed for microsatellites with length $\geq$ 8 bp (data not shown).

### Validation Set: case-control study

Following our observation that UL-MSI was detected in microsatellites $\geq$ 8 bp in length regardless of the loci, a second series of 45 samples was sequenced using an almost totally distinct panel of microsatellites that were $\geq$ 8 bp in length and included only 395 loci in common with the first study. In this second series of CMMRD cases (9 confirmed and 5 suspected) and MSS controls (healthy individuals, $N$=31), the ms.score for MSS controls ranged from 0 to 1 (mean=0.49), compared to 1.19 to 4.13 (mean=2.36) and 1.11 to 3.84 (mean=2.31) for confirmed and suspected CMMRD PBCs, respectively (Figure 2B). Comparison of the ms.score distributions between the MSS and confirmed CMMRD individuals showed a statistically significant difference ($P < 2.2x10^{-16}$), allowing again accurate discrimination of CMMRD samples from MSS controls with 100% sensitivity. As in the previous study, all the suspected CMMRD cases supported the CMMRD status previously diagnosed. Again, the critical inputs for the algorithms were the number of loci and the depth of coverage (data not shown). As expected, differences in the ms.score between confirmed CMMRD and MSS samples were significant for all selected microsatellites ($\geq$ 8 bp in length; see above and data not shown).

### Optimized probes for the detection of confirmed and suspected CMMRD individuals

Finally, we aimed to limit the number of probes in the panel that are required to distinguish CMMRD subjects from healthy MSS controls. A subset of the more discriminating probes was obtained by applying a threshold-filter to the distribution of this parameter. With the first panel used in the proof-of-concept study, only 118 loci were necessary to discriminate MSS from CMMRD or MSI positive controls as accurately as the full panel. Using this subset of loci, the ms.score for the MSS controls ranged from 0 to 1 (mean=0.47) as compared to 12.77 to 30.74 (mean=20.21) and 13.98 to 16.16 (mean=15.07) for confirmed and suspected cases of CMMRD, respectively (MSS controls vs confirmed CMMRD PBCs, P=0.007; see Figure 1C). With the second panel used in the case-control study and operating with the same two parameters as in the discovery study, just 18 loci could discriminate MSS from confirmed or suspected CMMRD cases as accurately as the full panel. The ms.score for MSS controls ranged from 0 to 1 (mean=0.49) as compared to 1.74 to 4.30 (mean=2.77) and 1.62 to 4.13 (mean=2.81) for confirmed and suspected CMMRD cases, respectively (MSS controls vs confirmed CMMRD PBCs, $P < 2.2x10^{-16}$) (Figure 1C').

### Classification CMMRS and MSI tumour samples in 2 assays

Two new assays were realized to confirm the capability of the method of the invention to correctly classify CMMRD and, in general, MSI tumour samples. The first with 15 non-tumoral MSS control samples, 27 CMMRD blood samples and 4 MSI colon cancer samples and the second with 10 non-tumoral MSS control samples, 6 CMMRD blood samples and 2 MSI colon cancer samples. Those 2 assays confirmed the capability of the proposed method to correctly classify CMMRD and, in general, MSI tumour samples. In both assays sensitivity and specificity were 100% (Fig 3 A and 3 B respectively)

## DISCUSSION

MSI testing of tumor DNA has recently emerged to be crucial for the identification of metastatic cancer patients who may benefit from immune checkpoint inhibitors [9]. Here we demonstrate that MSI testing of germline DNA can also be used to detect a major predisposition to the development of tumors in CMMRD patients and for whom successful treatment with immune checkpoint inhibitors was recently reported [4,5]. The test proposed here for the screening of CMMRD using PBCs is feasible and shows 100% sensitivity and specificity in our hands (Table 1). The implementation of this method would easily permit the identification of patients bearing UL-MSI, and thus affected by the CMMRD syndrome, owing to the respect of three critical parameters, *i.e.* an average depth of coverage ranging from 1000X to 5000X, and at least one hundred targeted mononucleotide repeats of 8 base pairs or more. Importantly, this test is much faster than the one we recently proposed and which requires to cultivate LCLs from patients [6]. In addition, it has a much greater sensitivity and specificity than the previous approaches that have been developed to identify germline MSI from the examination of patients' PBCs [10] (Table 1). We recommend its use for children with a tumor of the CMMRD spectrum in order to allow rapid diagnosis of the syndrome and before the occurrence of other tumors later in life.

## REFERENCES:

1. Lavoine N, Colas C, Muleris M, et al. Constitutional mismatch repair deficiency syndrome: clinical description in a French cohort. Journal of medical genetics. 2015;52(11):770-778.
2. Wimmer K, Kratz CP, Vasen HF, et al. Diagnostic criteria for constitutional mismatch repair deficiency syndrome: suggestions of the European consortium 'care for CMMRD' (C4CMMRD). Journal of medical genetics. 2014;51(6):355-365.
3. Vasen HF, Ghorbanoghli Z, Bourdeaut F, et al. Guidelines for surveillance of individuals with constitutional mismatch repair-deficiency proposed by the European Consortium "Care for CMMR-D" (C4CMMR-D). Journal of medical genetics. 2014;51(5):283-293.
4. Bouffet E, Larouche V, Campbell BB, et al. Immune Checkpoint Inhibition for Hypermutant Glioblastoma Multiforme Resulting From Germline Biallelic Mismatch Repair Deficiency. J Clin Oncol. 2016;34(19):2206-2211.
5. Larouche V, Atkinson J, Albrecht S, et al. Sustained complete response of recurrent glioblastoma to combined checkpoint inhibition in a young patient with constitutional mismatch repair deficiency. Pediatr Blood Cancer. 2018;65(12):e27389.
6. Bodo S, Colas C, Buhard O, et al. Diagnosis of Constitutional Mismatch Repair-Deficiency Syndrome Based on Microsatellite Instability and Lymphocyte Tolerance to Methylating Agents. Gastroenterology. 2015;149(4):1017-1029 e1013.
7. Jonchere V, Marisa L, Greene M, et al. Identification of Positively and Negatively Selected Driver Gene Mutations Associated With Colorectal Cancer With Microsatellite Instability. Cell Mol Gastroenterol Hepatol. 2018;6(3):277-300.
8. Hause RJ, Pritchard CC, Shendure J, Salipante SJ. Classification and characterization of microsatellite instability across 18 cancer types. Nature medicine. 2016;22(11):1342-1350.
9. Le DT, Uram JN, Wang H, et al. PD-1 Blockade in Tumors with Mismatch-Repair Deficiency. The New England journal of medicine. 2015;372(26):2509-2520.
10. Ingham D, Diggle CP, Berry I, et al. Simple detection of germline microsatellite instability for diagnosis of constitutional mismatch repair cancer syndrome. Human mutation. 2013;34(6):847-852.
11. Niu B, Ye K, Zhang Q, et al. MSIsensor: microsatellite instability detection using paired tumor-normal sequence data. Bioinformatics. 2014;30(7):1015-1016.

## Claims

1.  A method of diagnosing a Constitutional MisMatch Repair Deficiency (CMMRD) cancer or a MicroSatellite Instability (MSI) leukemia/lymphoma in a patient in need thereof comprising i) extracting DNA from a sample obtained from said patient ii) sequencing a number (N) of repeat sequences having a length of (x) nucleic acids from the DNA of said patient, iii) repeating the steps i) and ii) for at least one control subject having stable microsatellite cancer (Micro-Satellite Stability - or MSS - control subject), iv) doing a log 10 transformation of the reads counts per locus for said patient and for the MSS control subject(s), and doing a limit regression for each repeat obtained from the MSS control subject(s), and v) obtaining the ms.score by doing the following formula :

$$\sum_{n=1}^{N} \Delta_n$$

wherein, N = number maximal of repeat sequenced; n = number of repeat sequences, $\Delta$ = number of reads (patient in need thereof sample) - number of reads (limit regression from the MSS control subject(s)) and vi) comparing the ms.score obtained with the patient in need thereof with the ms.score of the MSS control subject(s) and vii) concluding that the patient in need thereof has a CMMRD cancer or a MSI leukemia/lymphoma when his ms.score is superior than the ms.score of the MSS control subject(s).

2. A method of diagnosing according to claim 1 wherein the sequencing is an ultra-deep sequencing like Second-Generation Sequencing (NGS).

3. A method according to claim 1 wherein the sequencing is done to a number of loci between 10 and 10000.

4. A method according to claim 1 wherein the number of reads is between 500 and 4000 and more particularly between 1000 and 4000.

5. A method according to claim 1 wherein the lengths (x) of the repeat of nucleic acids is between 8 and 30 and particularly 8 and 14.

6. A method according to claim 1 wherein the CMMRD cancer is a CMMRD colorectal cancer.

7. A method according to claim 1 wherein the number of MSS control(s) can be from 1 to 30.

8. A method according to claims 1 to 7 wherein the DNA is germinal DNA.


**Patentansprüche**

1. Verfahren zur Diagnose eines Constitutional-Mismatch-Reparatur-Defekt-, (CMMRD)-Krebses oder einer/eines Micro-Satellite-Instability-, (MSI)-Leukämie/Lymphoms bei einem bedürftigen Patienten, umfassend: i) Extrahieren von DNA aus einer Probe, die von dem Patienten erhalten wurde, ii) Sequenzieren einer Anzahl (N) von Wiederholungssequenzen, die eine Länge von (x) Nukleinsäuren aufweisen, aus der DNA des Patienten, iii) Wiederholen der Schritte i) und ii) für mindestens ein Kontrollsubjekt, das einen stabilen Mikrosatellitenkrebs (MicroSatellite Stability-oder MSS- Kontrollsubjekt) aufweist, iv) Durchführen einer Log-10-Transformation der Readcounts pro Stelle für den Patienten und für das/die MSS-Kontrollsubjekt(e), und Durchführen einer Grenzwertregression für jede Wiederholung, die von der/den MSS-Kontrollsubjekt(en) erhalten wurde, und v) Erhalten des MS-Werts durch Anwenden der folgenden Formel:

$$\sum_{n=1}^{N} \Delta_n$$

wobei N = maximale Anzahl der sequenzierten Wiederholungen; n = Anzahl der Wiederholungssequenzen, $\Delta$ = Anzahl der Reads (Probe des bedürftigen Patienten) - Anzahl der Reads (Grenzwertregression von dem/den MSS-Kontrollsubjekt/en) und vi) Vergleichen des mit dem bedürftigen Patienten erhaltenen MS-Werts mit dem MS-Werts des/der MSS-Kontrollsubjekt(e) und vii) Schlussfolgern, dass der bedürftige Patient an einem CMMRD-Krebs oder einer/einem MSI-Leukämie/Lymphom leidet, wenn sein MS-Wert höher ist als der MS-Wert des/der MSS-Kontrollsubjekts(e).

2. Verfahren zur Diagnose nach Anspruch 1, wobei das Sequenzieren ein Ultra-Deep Sequenzieren, wie Sequenzieren der zweiten Generation (NGS) ist.

3. Verfahren nach Anspruch 1, wobei das Sequenzieren an einer Anzahl von Stellen zwischen 10 und 10000 durchgeführt wird.

**4.** Verfahren nach Anspruch 1, wobei die Anzahl an Reads zwischen 500 und 4000 und insbesondere zwischen 1000 und 4000 liegt.

**5.** Verfahren nach Anspruch 1, wobei die Längen (x) der Wiederholung von Nukleinsäuren zwischen 8 und 30 und insbesondere zwischen 8 und 14 liegen.

**6.** Verfahren nach Anspruch 1, wobei der CMMRD-Krebs ein CMMRD-Darmkrebs ist.

**7.** Verfahren nach Anspruch 1, wobei die Anzahl der MSS-Kontrolle(n) von 1 bis 30 betragen kann.

**8.** Verfahren nach den Ansprüchen 1 bis 7, wobei die DNA Keimzellen-DNA ist.

**Revendications**

**1.** Méthode de diagnostic d'un cancer lié à un déficit constitutionnel de réparation des mésappariements (CMMRD) ou d'une leucémie/lymphome liée à une instabilité des microsatellites (MSI) chez un patient qui en a besoin, comprenant : i) extraire de l'ADN à partir d'un échantillon prélevé sur ledit patient ; ii) séquencer un nombre (N) de séquences répétées d'une longueur de (x) acides nucléiques à partir de l'ADN dudit patient ; iii) répéter les étapes i) et ii) pour au moins un sujet témoin présentant un cancer à microsatellites stables (sujet témoin à stabilité des microsatellites - ou MSS), iv) effectuer une transformation logarithmique en base 10 du nombre de lectures par locus pour ledit patient et pour le ou les sujet(s) témoin(s) MSS, et effectuer une régression limite pour chaque répétition obtenue à partir du ou des sujet(s) témoin(s) MSS, et v) obtenir le ms.score en appliquant la formule suivante :

$$\sum_{n=1}^{N} \Delta_n$$

dans laquelle N = nombre maximal de séquences répétées ; n = nombre de séquences répétées ; $\Delta$ = nombre de lectures (échantillon du patient concerné) - nombre de lectures (régression limite du ou des sujet(s) témoin(s) MSS) ; et vi) comparer le ms.score obtenu pour le patient qui en a besoin avec le ms. score du ou des sujet(s) témoin(s) MSS et vii) conclure que le patient qui en a besoin est atteint d'un cancer CMMRD ou d'une leucémie/lymphome MSI lorsque son ms.score est supérieur au ms.score du ou des sujet(s) témoin(s) MSS.

**2.** Méthode de diagnostic selon la revendication 1, dans laquelle le séquençage est un séquençage ultra-profond tel que le séquençage de deuxième génération (NGS).

**3.** Méthode selon la revendication 1, dans laquelle le séquençage porte sur un nombre de loci compris entre 10 et 10 000.

**4.** Méthode selon la revendication 1, dans laquelle le nombre de lectures est compris entre 500 et 4 000, et plus particulièrement entre 1 000 et 4 000.

**5.** Méthode selon la revendication 1, dans laquelle les longueurs (x) de la répétition des acides nucléiques sont comprises entre 8 et 30, et plus particulièrement entre 8 et 14.

**6.** Méthode selon la revendication 1, dans laquelle le cancer CMMRD est un cancer colorectal CMMRD.

**7.** Méthode selon la revendication 1, dans laquelle le nombre de contrôle(s) MSS peut être compris entre 1 et 30.

**8.** Méthode selon les revendications 1 à 7, dans laquelle l'ADN est de l'ADN germinal.

# MMR Proficient Cell

Figure 1 A

# MMR Deficient Primary Blood Cell (PBC)

Detection of Ultra low MSI

**Functional Evidence:**
- dMMR → MSI → Cancer

**Hypothesis:**
- MSI is present at ultra-low levels (UL-MSI) in dMMR PBCs from CMMRD patients prior to cell transformation
- UL-MSI is detectable by Massive Parallel Sequencing

**Figure 1 A'**

Figure 1 B

**ms.score**

$$\sum_{n=1}^{N} \Delta_n$$

where :

Δ = Nb. of reads (MSI or CMMRD sample) - nb. of reads (MSS controls)

**ms.score in        ms.score in        ms.score in
MSI cancer >> CMMRD PBC >> MSS controls**

**Figure 1 B'**

**Figure 1C**

Figure 1 C'

Figure 2 A

Figure 2 B

Figure 3A

## ms.score (1488 loci max)

Figure 3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070254295 A
- US 5618829 A
- US 5639757 A
- US 5728868 A
- US 5804396 A
- US 6100254 A
- US 6127374 A
- US 6245759 B
- US 6306874 B
- US 6313138 B
- US 6316444 B
- US 6329380 B
- US 6344459 B
- US 6420382 B
- US 6479512 B
- US 6498165 B
- US 6544988 B
- US 6562818 B
- US 6586423 B
- US 6586424 B
- US 6740665 B
- US 6794393 B
- US 6875767 B
- US 6927293 B
- US 6958340 B

**Non-patent literature cited in the description**

- PCR.. *Gastroenterology.*, 2002
- **BUHARD O et al.** Multipopulation analysis of polymorphisms in five mononucleotide repeats used to determine the microsatellite instability status of human tumors.. *J Clin Oncol.*, 2006
- **MAXAM-GILBERT**. *Methods in Enzymology*, 1980, vol. 65, 499-560
- **SANGER**. *Proc. Natl. Acad. Sci. USA*, 1977, vol. 74, 5463-67
- **TABOR et al.** *Proc. Natl. Acad. Scl. USA*, 1987, vol. 84, 4767-4771
- **GOODWIN, S**. Coming of age: Ten years of next-generation sequencing technologies.. *Nature Reviews Genetics*, 2016
- *Agnew Chem Intl. Ed. Engl.*, 1994, vol. 33, 183-186
- **NEGRIER et al.** *Ann Oncol.*, 2002, vol. 13 (9), 1460-8
- **TOURANIETAL**. *J. Clin. Oncol.*, 2003, vol. 21 (21), 398794
- **NICHOLAS P. RESTIFO** ; **MARK E. DUDLEY** ; **STEVEN A. ROSENBERG**. Adoptive immunotherapy for cancer: harnessing the T cell response. *Nature Reviews Immunology*, April 2012, vol. 12
- **LAVOINE N** ; **COLAS C** ; **MULERIS M et al.** Constitutional mismatch repair deficiency syndrome: clinical description in a French cohort.. *Journal of medical genetics.*, 2015, vol. 52 (11), 770-778
- **WIMMER K** ; **KRATZ CP** ; **VASEN HF et al.** Diagnostic criteria for constitutional mismatch repair deficiency syndrome: suggestions of the European consortium 'care for CMMRD' (C4CMMRD).. *Journal of medical genetics.*, 2014, vol. 51 (6), 355-365
- **VASEN HF** ; **GHORBANOGHLI Z** ; **BOURDEAUT F et al.** Guidelines for surveillance of individuals with constitutional mismatch repair-deficiency proposed by the European Consortium ''Care for CMMR-D'' (C4CMMR-D).. *Journal of medical genetics.*, 2014, vol. 51 (5), 283-293
- **BOUFFET E** ; **LAROUCHE V** ; **CAMPBELL BB et al.** Immune Checkpoint Inhibition for Hypermutant Glioblastoma Multiforme Resulting From Germline Biallelic Mismatch Repair Deficiency.. *J Clin Oncol.*, 2016, vol. 34 (19), 2206-2211
- **LAROUCHE V** ; **ATKINSON J** ; **ALBRECHT S et al.** Sustained complete response of recurrent glioblastoma to combined checkpoint inhibition in a young patient with constitutional mismatch repair deficiency.. *Pediatr Blood Cancer.*, 2018, vol. 65 (12), e27389
- **BODO S** ; **COLAS C** ; **BUHARD O et al.** Diagnosis of Constitutional Mismatch Repair-Deficiency Syndrome Based on Microsatellite Instability and Lymphocyte Tolerance to Methylating Agents.. *Gastroenterology.*, 2015, vol. 149 (4), 1017-1029

- **JONCHERE V** ; **MARISA L** ; **GREENE M et al.** Identification of Positively and Negatively Selected Driver Gene Mutations Associated With Colorectal Cancer With Microsatellite Instability.. *Cell Mol Gastroenterol Hepatol.*, 2018, vol. 6 (3), 277-300
- **HAUSE RJ** ; **PRITCHARD CC** ; **SHENDURE J** ; **SALIPANTE SJ.** Classification and characterization of microsatellite instability across 18 cancer types.. *Nature medicine.*, 2016, vol. 22 (11), 1342-1350
- **LE DT** ; **URAM JN** ; **WANG H et al.** PD-1 Blockade in Tumors with Mismatch-Repair Deficiency.. *The New England journal of medicine.*, 2015, vol. 372 (26), 2509-2520
- **INGHAM D** ; **DIGGLE CP** ; **BERRY I et al.** Simple detection of germline microsatellite instability for diagnosis of constitutional mismatch repair cancer syndrome.. *Human mutation.*, 2013, vol. 34 (6), 847-852
- **NIU B** ; **YE K** ; **ZHANG Q et al.** MSIsensor: microsatellite instability detection using paired tumor-normal sequence data.. *Bioinformatics.*, 2014, vol. 30 (7), 1015-1016